# EUROPEAN PATENT APPLICATION

(11) **EP 3 318 628 A1**
(43) Date of publication of application: **09.05.2018**
(21) Application number: 16315010.5
(22) Date of filing: 08.11.2016
(51) Int. Cl.: C12N 5/077, C07K 14/47

(54) **COMPONENTS AND METHODS FOR INDUCING SKELETAL MYOGENIC DIFFERENTIATION**

(71) Applicant: CNRS Centre National de la Recherche Scientifique, 75016 Paris (FR); UNIVERSITE DE NANTES, 44000 Nantes (FR)
(72) Inventor: Thiriet, Christophe, 44322 Nantes (FR); Rouaud, Thierry, 44322 Nantes (FR); Boucher, Gwenola, 44322 Nantes (FR); Gervier, Pascal, 44322 Nantes (FR)
(74) Representative: Belbeoc'h, Stéphanie

(57) **Abstract**

The present invention relates to a factor, in particular a complement protein C3, useful for inducing the differentiation of myogenic progenitor cells into myogenic cells and its applications.

## Description

The present invention relates to the field of skeletal myogenic differentiation.

In particular, the invention relates to a factor useful for inducing the differentiation of skeletal myogenic progenitor cells into skeletal myogenic cells.

Skeletal myogenic cell differentiation is a complex process involving the activation, proliferation and differentiation of skeletal myogenic progenitor cells. During development and regeneration, skeletal myogenic differentiation is perfectly coordinated and proceeds to form and regenerate the skeletal muscles. The overall cellular mechanism consisting in the proliferation and the fusion of the skeletal myogenic progenitor cells leading to multinucleated syncytia is shared throughout the life span. Upon activation, skeletal myogenic progenitor cells express specific myogenic transcription factors, such as MyoD, Myf5 and Myogenin. This genetic cascade leads to the differentiation into skeletal myogenic cells that generates new muscle fibers.

However, the factors able to activate skeletal progenitor cells to induce skeletal myogenic differentiation are still not well characterized. Such factors could be useful for therapeutic but also for *ex vivo* applications. In fact, in the conduct of certain cell biology research, for example to study skeletal muscle cells disorders, the myogenic differentiation of cells in culture is essential.

Some media have been described and are commercialized for the differentiation of skeletal muscle cells *in vitro.* However, some of these media have not a strong effect on skeletal myogenic differentiation. Some others contain a lot of compounds and are thus not appropriate for cell biology and/or biochemical studies, in particular of skeletal muscle cells disorders.

Thus, there remains a significant need for new and improved factors which are highly effective for inducing the differentiation of skeletal myogenic progenitor cells into skeletal myogenic cells, while being simple and not expensive. The inventors have made a significant step forward with the invention disclosed herein.

The purpose of the invention is to fulfil this need by providing new skeletal myogenic differentiation factors, which make it possible to solve in whole or part the problems mentioned above.

Unexpectedly, the inventors have demonstrated that the complement protein C3, a key factor of the innate immune system (comprised or not in a preadipocytes conditioned medium) is able to promote the differentiation of skeletal myogenic progenitor cells into skeletal myogenic cells, following its rapid internalisation within the cytoplasm.

In one aspect, the invention relates to an *ex vivo* method for inducing the differentiation of skeletal myogenic progenitor cells into skeletal myogenic cells, said method comprising the step of:
- culturing the skeletal myogenic progenitor cells in the presence of a complement protein C3.

The *ex vivo* method according to the invention has in particular the following advantages:
- It has a strong effect on myogenic cell differentiation, up to 2.5 times better than other tested commercial media;
- It is not expensive, quick and easy to implement;
- It is appropriate for biochemical studies of muscle cell disorders, in particular not requiring a lot of factors but the complement protein C3.
- It used a natural molecule.

Unless defined otherwise, all technical and scientific terms used herein have the same meaning as commonly understood to one skilled in the relevant art.

For convenience, the meaning of certain terms and phrases employed in the specification, examples and claims are provided.

As used herein, the term "skeletal myogenic progenitor cells" refers to any cells which could evolve into myogenic cells by differentiation. The ability of a cell to differentiate into a skeletal myogenic cells can be evaluated according to any techniques well-known to one skilled in the art, including but not limiting to the detection of at least one specific marker of skeletal myogenic cells such as myosin (as described in the examples below), after the implementation of the *ex vivo* method of the invention. In particular, skeletal myogenic progenitor cells can be satellite cells or stem cells. For example, myogenic progenitor cells can express specific transcription factors, such as MyoD, Myf5 and Myogenin.

As used herein, the term "skeletal myogenic cells" refers to any differentiated myogenic cells. Skeletal myogenic cells can be identified according to any techniques well-known to one skilled in the art, including but not limiting to the detection of at least one specific marker of skeletal myogenic cells such as myosin, tropomyosins and troponins.

As used herein, the term "complement protein C3" refers to the third component of the complement system. Complement protein C3 is a central molecule in the complement system whose activation is essential for all the important functions performed by this system as described in the article of Sahu, A. & Lambris, J.D (2001). For example, the wild type homo sapiens complement protein C3 is a 1663 amino-acids protein (referenced: NCBI protein ID# AAA85332.1; UniProtKB/Swiss-Prot Prot ID# P01024). The chromosome location of the human gene encoding the human complement protein C3 is 19p13.3. Purified, in particular human, complement protein C3 can be purchased from different companies such as Biorad (product code: PHP283), Sigma Aldrich (product code: C2910) or Tecomedical (product code: A401). The term "complement protein C3" as used herein encompasses natural variants of the complement protein C3 as described above but also peptidomimetic variants of the complement protein C3 having modified amino acid sequences. Modifications can include but are not limited to amino acid insertions, deletions, substitutions, truncations, fusions, cyclization, disulphide bridging, substitution of D-aminoacids by L-amino acids or by beta peptides, modifications to improve cell-membrane pass-through provided that the peptidomimetic variants of the complement protein C3 retain the ability to induce the differentiation of myogenic progenitor cells into myogenic cells. Such modifications may be undertaken to improve biological activity or reduce the size of the protein, in particular in case the *ex vivo* method of the invention comprising a transfection step as described below. In particular, the differentiation effect of the variants derivatives of a wild type complement protein C3 on myogenic progenitor cells has to be of at least about 70%, preferably between 80% and 90%, more preferably between 90% and 99%, and even more preferably 100% of the differentiation effect of the parent wild type complement protein C3 (in particular referenced: NCBI protein ID# AAA85332.1; UniProtKB/Swiss-Prot Prot ID# P01024).

In particular, the species of the complement protein C3 used according to the invention is the same as the species of the skeletal myogenic progenitor cells. Particularly, the sequence of the complement protein C3 is the sequence of the wild type human complement protein C3 (NCBI protein ID# AAA85332.1; UniProtKB/Swiss-Prot Prot ID# P01024), when using human skeletal myogenic progenitor cells.

The ability of a factor to induce the differentiation of skeletal myogenic progenitor cells into skeletal myogenic cells can be evaluated according to any techniques well-known to one skilled in the art, including but not limiting to the detection (qualitatively and advantageously quantitatively such as by immunocytochemistry, immunoblotting...) of at least one specific marker of skeletal myogenic cells such as myosin (as described in the examples below), after the implementation of the *ex vivo* method of the invention wherein the complement protein C3 is replaced by said factor to be tested. A wild-type complement protein C3 can be used as a positive control.

According to the *ex vivo* method of the invention, the skeletal myogenic progenitor cells can be cultured in the presence, intra-cellular and/or extracellular, of a complement protein C3. In fact, the inventors have demonstrated that complement protein C3 internalizes within the cytoplasms of skeletal myogenic precursor cells and then induces their differentiation into myogenic cells.

In particular, the *ex vivo* method according to the invention further comprises the step of:
- transfecting the skeletal myogenic progenitor cells with an expression vector encoding a complement protein C3.

The step of transfecting the skeletal myogenic progenitor cells can be implemented by any techniques well-known to one skilled in the art, including but not limiting to reagent-based methods using liposome, calcium-phosphate, cationic polymers, DEAE-dextran, activated dendrimers or magnetic beads, instrument-based methods using electroporation, biolistic technology, microinjection or laserfection/optoinjection and virus-based methods.

As used herein the term "expression vector encoding a complement protein C3" refers to an expression vector comprising a nucleic acid molecule encoding a complement protein C3. Said vector can be appropriated for semi-stable or stable expression. The vectors can be viral vectors such as bacteriophages or non-viral such as plasmids.

In particular, said step of transfecting the skeletal myogenic progenitor cells can be performed before the step of culturing the skeletal myogenic progenitor cells in the presence, intracellular and/or extracellular, more particularly intracellular, of a complement protein C3.

In one embodiment of the *ex vivo* method according to the invention, said complement protein C3 is comprised in a preadipocytes conditioned medium. The step of culturing the skeletal myogenic progenitor cells can be performed in the presence of, particularly in, a preadipocytes conditioned medium comprising a complement protein C3.

As used herein the term "preadipocytes conditioned medium" refers to a medium prepared from the supernatant of a proliferative adipogenic lineage culture before preadipocytes begin to differentiate into adipocytes. Such medium can be prepared according to any techniques well-known by one skilled in the art, including but not limiting to the method described below in the Examples. In particular, said preadipocytes conditioned medium can be prepared from the supernatant of 24h of preadipocytes cell culture between day 3-4. Two successive passages can be performed in a similar manner to collect two other different preadipocytes conditioned medium. The supernatants can be filtered (particularly upon 0.22 µm).

In one embodiment, the preadipocytes conditioned medium can be mixed to another culture medium, before or during the step of culturing the skeletal myogenic progenitor cells.

The step of culturing the skeletal myogenic progenitor cells can be implemented by any techniques well known to one skilled in the art, including but not limiting to culturing cells in a culture medium, in particular on collagen-coated surface as described in the examples below. Said culture medium can be any medium which is appropriate for culturing the skeletal myogenic progenitor cells and/or the skeletal myogenic cells. In particular, said culture medium can be a basal medium for culturing cells, in particular mammalian cells. The term "basal medium" refers to an unsupplemented growth medium, which promotes the growth of cells. The term "basal medium for culturing mammalian cells" refers to an unsupplemented growth medium, which promotes the growth of mammalian cells. For example, said basal medium can be a Dulbecco's modified Eagle's medium (e.g. ThermoFisher scientific 41966) or RPMI (ThermoFisher scientific 1640). Both said exemplified basal media are also for culturing mammalian cells. More particularly, said culture medium can be a basal medium supplemented with a decomplemented serum. For example, said culture medium can be Dulbecco's modified Eagle's medium (DMEM) supplemented with decomplemented foetal calf serum (in particular 10%).

In one embodiment of the *ex vivo* method according to the invention, said complement protein C3 is comprised in a culture medium, which further comprises at least another compound selected from the group consisting of:
- antibiotics, amino-acids, vitamins, inorganics salts, carbohydrates, hormones, cytokines, growth factors and skeletal myogenic differentiation factors, in particular growth factors.

For example, antibiotics can be chosen in the group consisting of actinomycin D, ampicillin, carbenicillin, cefotaxime, fosmidomycin, gentamicin, kanamycin, neomycin, penicillin, polymyxin B, streptomycin and amphotericin B. In particular, said culture medium can comprise at least 2 and more particularly at least 3 different antibiotics such as penicillin, streptomycin and amphotericin B.

For example, aminoacids can be chosen in the group consisting of Glycine, L-Arginine hydrochloride, L-Cystine 2HCl, L-Glutamine, L-Histidine hydrochloride-H2O, L-isoleucine, L-Leucine, L-Lysine hydrochloride, L-Methionine, L-Phenylalanine, L-Serine, L-Threonine, L-Tryptophan, L-Tyrosine disodium salt dihydrate and L-Valine. In particular, said culture medium can comprise at least 2, at least 3, at least 4, at least 5, at least 6, at least 7, at least 8 ; at least 9, at least 10, at least 11, at least 12, at least 13, at least 14 and more particularly at least 15 different amino-acids.

For example, vitamins can be chosen in the group consisting of Choline chloride, D-Calcium pantothenate, Folic Acid, Niacinamide, Pyridoxine hydrochloride, Riboflavin, Thiamine hydrochloride and i-Inositol. In particular, said culture medium can comprise at least 2, at least 3, at least 4, at least 5, at least 6, at least 7 and more particularly at least 8 different vitamins.

For example, inorganics salts can be chosen in the group consisting of Calcium Chloride, Ferric Nitrate, Magnesium Sulfate, Potassium Chloride, Sodium Bicarbonate, Sodium Chloride and Sodium Phosphate monobasic. In particular, said culture medium can comprise at least 2, at least 3, at least 4, at least 5, at least 6 and more particularly at least 7 different inorganics salts.

In particular, carbohydrates can be D-Glucose.

In particular, hormones can be dexamethasone.

In particular, cytokines can be Interleukin (IL)-6.

In particular, said culture can also comprise some phenol red.

In particular, growth factors comprised in said culture medium can be myogenic growth factors. For example, said growth factors can be chosen in the group consisting of insulin, insulin-like growth factors in particular IGF-1, hepatocyte growth factor, epidermal growth factor, vascular endothelial cell growth factor (VEGF), transforming growth factor in particular TGF-β, fibroblast growth factors in particular FGF-1 and FGF-2, thrombospondins in particular TSP-1, the sonic hedgehog protein (Shh), angiopetins in particular Ang-1 and Ang-2, the Hh factors, platelet-derived growth factor (PDGF), hepatocyte growth factor (HGF).

In one embodiment of the *ex vivo* method according to the invention, in the culture medium, the concentration of said complement protein C3 ranges from 0,05 % to 2 % in particular from 0,25 % to 1,75 % and more particularly from 0,5 % to 1,25 % and even more particularly 1 %.

The invention also related to an *ex vivo* use of a complement protein C3 or a nucleic acid molecule encoding said protein as a skeletal myogenic differentiation factor.

As used herein, the term "skeletal myogenic differentiation factor" means any compound able to induce the differentiation of skeletal myogenic progenitor cells into skeletal myogenic cells. As described above, the ability of a factor to induce the differentiation of skeletal myogenic progenitor cells into skeletal myogenic cells can be evaluated according to any techniques well-known to one skilled in the art, including but not limiting to the detection (qualitatively and advantageously quantitatively such as by immunocytochemistry, immunoblotting...) of at least one specific marker of skeletal myogenic cells such as myosin (as described in the examples below), after the implementation of the *ex vivo* method of the invention wherein the complement protein C3 is replaced by said factor to be tested. A wild-type complement protein C3 can be used as a positive control.

In particular, said nucleic acid molecule encoding said complement protein C3 can be included in a vector, in particular an expression vector as described above. The advantageous embodiments are as defined above.

The invention also related to the use of a complement protein C3 or a nucleic acid molecule encoding said protein for the manufacture of a skeletal myogenic differentiation product, in particular a skeletal myogenic differentiation medium.

As used herein, the term "skeletal myogenic differentiation product" refers to any product able to induce the differentiation of myogenic progenitor cells into myogenic cells.

As used herein, the term "skeletal myogenic differentiation medium" refers to any culture medium able to induce the differentiation of myogenic progenitor cells into myogenic cells.

The advantageous embodiments are as defined above.

The invention also relates to an *ex vivo* use of an additive to a culture medium for inducing the differentiation of skeletal myogenic progenitor cells into skeletal myogenic cells, said additive comprising a complement protein C3.

As used herein, the term "additive" refers to any supplement added to a culture medium, in particular to a basal medium.

In particular, said additive further comprises at least another compound selected from the group consisting of:
- antibiotics, amino-acids, vitamins, inorganics salts, carbohydrates, hormones, cytokines, growth factors and skeletal myogenic differentiation factors.

In particular, said additive is serum-free, more particularly complemented serum-free. As used herein, the term "serum-free" means no presence of a serum, in particular no presence of foetal calf or bovine serum, decomplemented or not (i.e. complemented). In the embodiment wherein the additive according to the invention is complemented serum-free, said additive does not comprise any serum which has not been decomplemented but the additive can further comprise decomplemented serum. Decomplemented serum can be obtained according to any techniques well-known to one skilled in the art, including but not limiting to heating the serum, in particular at 55-56°C during 30 minutes, to inactivate complement proteins.

The advantageous embodiments are as defined above.

The invention also relates to a skeletal myogenic differentiation medium comprising, in particular consisting of:
- a basal medium, in particular for culturing mammalian cells; and
- a complement C3 protein, in particular a mammalian complement C3 protein.

In particular, the concentration of said complement protein C3 in said skeletal myogenic differentiation medium according to the invention ranges from 0,05 % to 2,25 % in particular from 0,25 % to 1,96 % and more particularly from 0,5 % to 1,4 % and even more particularly from 1% to 1,2 %. The advantageous embodiments are as defined above.

In one embodiment, the skeletal myogenic differentiation medium according to the invention is serum-free. In said embodiment, decomplemented serum (in particular 10%), such as decomplemented foetal calf or bovine serum, can be added to the skeletal myogenic differentiation medium according to the invention before using.

In another embodiment, the skeletal myogenic differentiation medium according to the invention can further comprise at least another compound selected from the group consisting of: antibiotics, hormones, cytokines, growth factors and skeletal myogenic differentiation factors.

In another embodiment, the skeletal myogenic differentiation medium according to the invention is complemented serum-free. In said embodiment, said skeletal myogenic differentiation medium does not comprise any serum which has not been decomplemented but said medium can further comprise a decomplemented serum, such as decomplemented foetal calf or bovine serum. For example, in said embodiment, the skeletal myogenic differentiation medium according to the invention can comprise Dulbecco's modified Eagle's medium (DMEM) (in particular 89%) supplemented with decomplemented foetal calf or bovine serum (in particular 10%) and a complement protein C3 (in particular 1 %).

The advantageous embodiments are as defined above.

The invention also relates to a skeletal myogenic differentiation kit comprising, in particular consisting of:
- a basal medium, in particular for culturing mammalian cells; and
- an additive comprising a complement protein C3, in particular a mammalian complement protein C3, wherein said additive is serum-free, in particular complemented serum-free.

For example, said basal medium for culturing mammalian cells can be a Dulbecco's modified Eagle's medium (e.g. ThermoFisher scientific 41966) or RPMI (ThermoFisher scientific 1640).

In particular, said skeletal myogenic differentiation kit according to the invention further comprises at least 1 antibiotic, in particular at least 2 and more particularly at least 3 antibiotics. The advantageous embodiments are as defined above.

In particular, said skeletal myogenic differentiation kit according to the invention further comprises a serum such as foetal calf or bovine serum. The advantageous embodiments are as defined above.

The invention also relates to an *ex vivo* use of the skeletal myogenic differentiation medium according to the invention or of the skeletal myogenic differentiation kit according to the invention, for inducing the differentiation of skeletal myogenic progenitor cells into skeletal myogenic cells. The advantageous embodiments are as defined above.

The invention also relates to a complement protein C3 or an acid nucleic molecule encoding said protein for use as a medicament in the treatment and/or prevention of a skeletal muscle loss condition.

As used herein the term "skeletal muscle loss condition" refers to any conditions wherein a muscle loss is a symptom. In particular, skeletal muscle loss conditions can be selected from the group consisting of:
- skeletal muscle degenerative diseases, in particular skeletal neuro-muscular degenerative diseases. More particularly, skeletal muscle degenerative diseases are selected from the group consisting of progressive muscular dystrophy, congenital muscular dystrophy, limb-girdle muscular dystrophy, facioscapulohumeral muscular dystrophy, myotonic muscular dystrophy, myopathy;
- skeletal muscular atrophy diseases, in particular neuromuscular atrophy diseases. More particularly, skeletal muscular atrophy diseases are selected from the group consisting of amyotrophic lateral sclerosis and spinal muscular atrophy.
- surgery-related muscle loss conditions; and
- age-related muscle loss conditions, in particular sarcopenia.

Skeletal muscle loss conditions can also occur after an accident.

In one embodiment, said skeletal muscle loss condition is selected from the group consisting of skeletal muscle degenerative diseases and skeletal muscular atrophy diseases.

Said complement protein C3 or acid nucleic molecule encoding said protein can be present in a medicament according to the invention in a therapeutically effective amount (active and non-toxic amount). A therapeutically effective amount refers to that amount of compound which ameliorates the symptoms or condition. Therapeutic efficacy and toxicity may be determined by standard pharmaceutical procedures in cell cultures or experimental animals, e.g., ED50 (the amount therapeutically effective in 50% of the population) and LD50 (the dose lethal to 50% of the population). The amount ratio of toxic to therapeutic effects is the therapeutic index and it can be expressed as the ratio, LD50/ED50. Medicaments which exhibit large therapeutic indices are preferred.

For example, said complement protein C3 according to the invention, can be administered to a patient, in particular by injection, in an amount within the range from 0.1 to 100 mg/kg of body weight of said patient daily, particularly within the range from 0.5 to 50 mg/kg of body weight of said patient daily and even more particularly within the range from 0.5 to 10 mg/kg of body weight of said patient daily. Said doses will be adjusted to elicit a therapeutic response.

The medicament according to the invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal means.

The invention also relates to a method for the treatment and/or prevention of a skeletal muscle loss condition comprising the step of administering to a subject in need thereof a therapeutically effective amount of at least one compound selected from the group consisting of a complement protein C3 or an acid nucleic molecule encoding said protein according to the invention. The advantageous embodiments are as defined above.

In particular, said subject can be a mammal, preferably a mouse, a rat, a cat, or a dog, and more preferably a human being.

The invention also relates to a combination product comprising:
- at least one complement protein C3 or one acid nucleic molecule encoding said protein; and
- at least one growth factor, in particular selected from the group consisting of myogenic growth factors and angiogenic growth factors,
for simultaneous, separate or sequential use a medicament.

As used herein, the term "angiogenic growth factor" refers to any protein, polypeptide, or portion thereof that is able of inducing angiogenic cell growth.

For example, said growth factors can be chosen in the group consisting of insulin-like growth factors in particular IGF-1, hepatocyte growth factor, vascular endothelial cell growth factor (VEGF), transforming growth factor in particular TGF-β, fibroblast growth factors in particular FGF-1 and FGF-2, thrombospondins in particular TSP-1, the sonic hedgehog protein (Shh), angiopetins in particular Ang-1 and Ang-2, the Hh factors, platelet-derived growth factor (PDGF), hepatocyte growth factor (HGF).

The invention also relates to a combination product according to the invention for its use in the treatment and/or prevention of a skeletal muscle loss condition. The advantageous embodiments are as defined above.

### BRIEF DESCRIPTION OF DRAWINGS

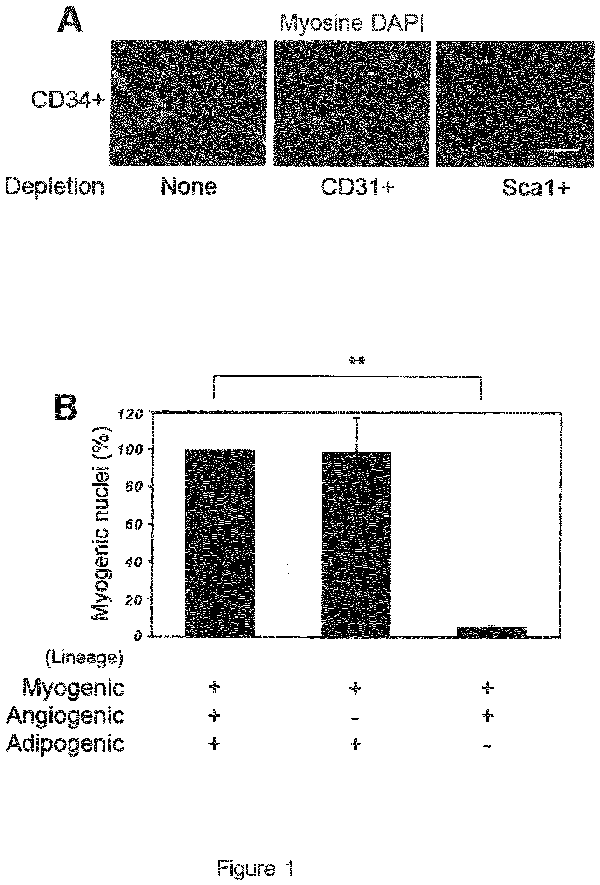

**Figure 1** **illustrates that adipogenic lineage cells are required for differentiation of myogenic progenitor cells into myogenic cells. a)** Microscopic observations of the myogenic differentiation of competent cells. Cells harvested from leg muscles of 17 dpc foetuses and sorted into a CD34⁺ population (CD34+/depletion none), CD34⁺ /CD31⁻ cells (CD34+/depletion CD31+), and CD34⁺ /Sca1⁻ cells (CD34+/depletion Sca1+), respectively. The different cell groups were cultured for 4 days and myogenic differentiation was assessed by immuno-staining using an anti-myosin antibody (My32, red), counterstained with DAPI (blue). Bar scale corresponds to 100 µm. **b)** Quantitative analyses of myogenic differentiation of competent cells. The percentage of myogenic differentiation was determined microscopically. Total CD34⁺ cell population was used as a reference and myogenic differentiation was arbitrarily estimated at 100%. Statistics: n=3, t-test P<0.0001 (**).

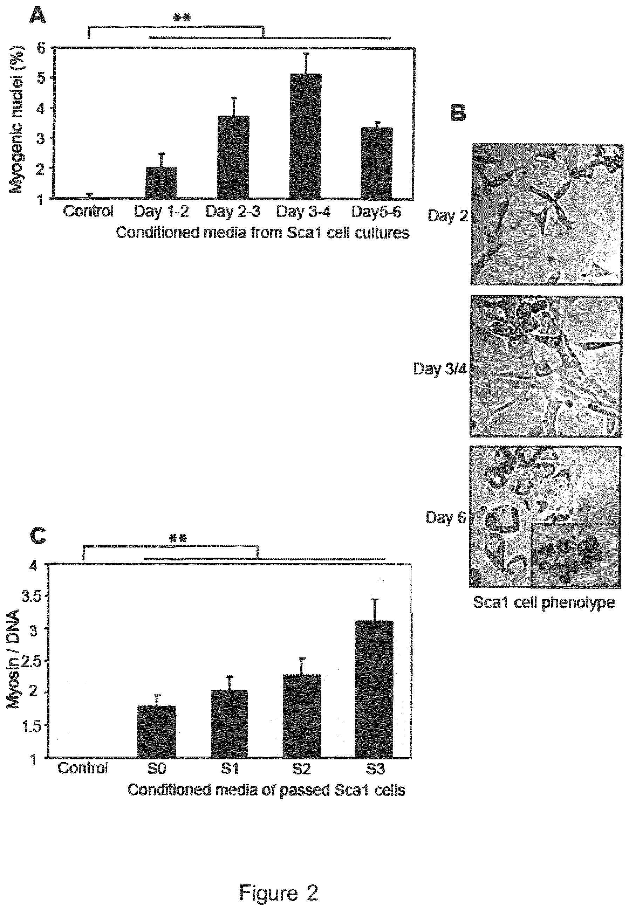

**Figure 2** **shows that proliferative preadipocytes produce myogenic differentiation factors.** a) Conditioned media prepared from CD34+/Sca1+ primary cell cultures enhanced myogenic differentiation of initial fraction cells. Conditioned media were prepared from 24 h of CD34+/Sca1+ cell cultures between day 1-2 (Day 1-2), day 2-3 (Day 2-3), day 3-4 (Day 3-4) and day 5-6 (Day 5-6), respectively. The percentage of myogenic nuclei was assessed microscopically on initial fraction cells cultured in conditioned media for 4 days, using cells in DMEM/decomplemented FCS as a control (Control). **b)** CD34+/Sca1+ cell phenotype in cell culture. Isolated CD34+/Sca1+ cells (Sca1) were cultured in DMEM/decomplemented FCS and micrographs were taken after 2 days (Day 2), 3-4 days (Day 3/4) and 6 days (Day 6), respectively. Insert in (Day 6) represents Oil Red O staining of differentiated adipocytes. c) Conditioned media from proliferative CD34+/Sca1+ cells enhanced myogenic differentiation. CD34+/Sca1+ primary cells were cultured and passaged just prior to confluence. Conditioned media were prepared between day 3 and 4 of primary cell culture (S0), after 1 passage (S1), after 2 passages (S2), and after 3 passages (S3), respectively. The myogenic differentiation was estimated by fluorescence (see details in materials and methods). Statistics: n=3, t-test P<0.0001 (**).

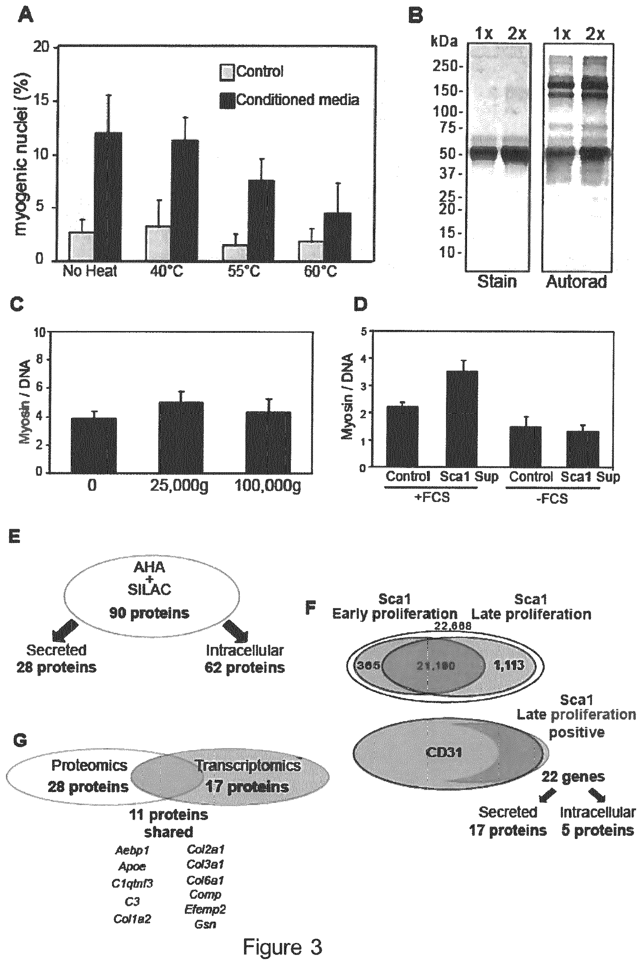

**Figure 3** **illustrates the Identification of the putative myogenic differentiation factors produced by undifferentiated adipocytes (i.e. preadipocytes). a)** Myogenic factors of the conditioned media are thermo-sensitive. Control and conditioned media were incubated at different temperatures for 15 min. The myogenic potential of the media was evaluated microscopically on the in initial fraction. **b)** CD34+/Sca1+ cells secrete proteins. CD34+/Sca1+ cells were pulsed with tritiated lysine for 24 h and the culture media were analyzed by SDS-PAGE (Stain) and autoradiography (Autorad). c) Myogenic factors secreted by CD34+/Sca1+ + cells are soluble. Conditioned media were centrifuged at 25,000 g and 100,000 g, respectively. The myogenic potential of the resulting media was then evaluated using non-centrifuged conditioned medium as a control (0). **d)** CD34+/Sca1+ cell cultures require FCS to produce efficient conditioned media. Conditioned media were prepared from CD34+/Sca1+ cultured in DMEM/decomplemented FCS (+FCS) and DMEM (-FCS), respectively. The myogenic potential of the resulting conditioned media was then evaluated using as control DMEM/decomplemented FCS (+FCS) and DMEM (-FCS), respectively. **e)** Proteomic analyses of CD34+/Sca1+ primary cell conditioned media. Proteins were prepared as described in Methods, identified by mass spectrometry and sorted by their cellular location; extracellular (Secreted) and intracellular (Intracellular). **f)** Transcriptomic analyses of CD34+/Sca1+ primary cells. Transcriptomes of CD34+/Sca1+ cells were analyzed in early proliferative state (lower efficiency of the secretome, lower rate of proliferation, Sca1 Early proliferation) and late proliferative state (higher efficiency of the secretome, higher rate of proliferation, Sca1 Late proliferation), respectively. The positive probes were sorted by the level of expression in the two culture states, arbitrary considering 2-fold changes as over-expression. The probes with higher expression in proliferative state were then analyzed against the transcriptome of CD34+/CD31+. The over-expression in CD34+/Sca1+ cells (Sca1 Late proliferation positive) relative to CD43+/CD31+ cells (CD31) was more than a 2-fold change. The gene products were then sorted by their cellular location; extracellular (Secreted) and intracellular (Intracellular). **g)** Cross-analyses of proteomics and transcriptomics revealed putative myogenic candidates. Comparison of secreted proteins from proteomic and transcriptomic analyses revealed 11 proteins in common.

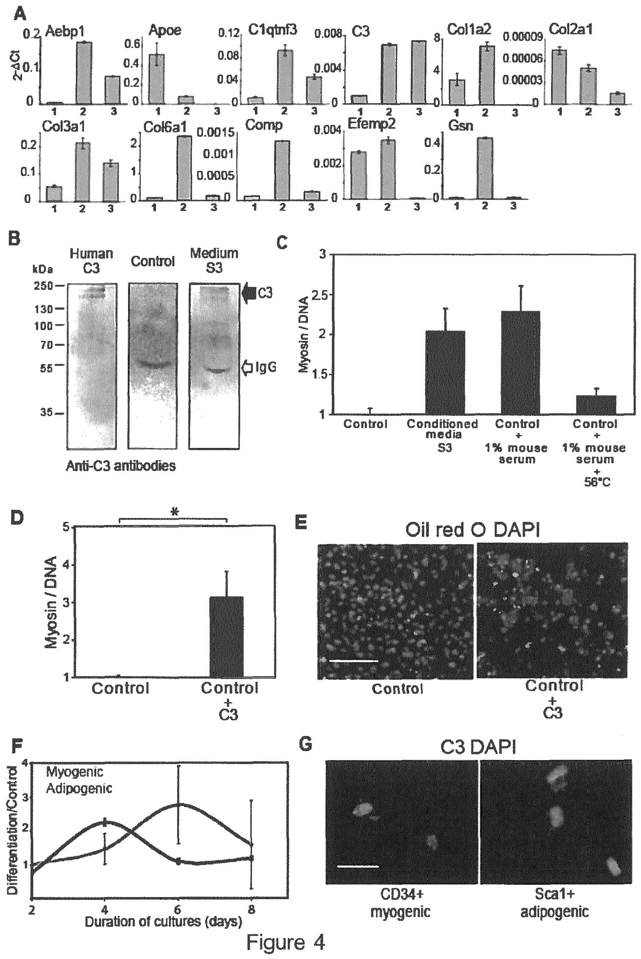

**Figure 4** **shows that complement protein C3 initiates cell differentiation of myogenic lineage cells and adipogenic lineage cells.** a) Expression of the putative myogenic factors in distinct cell cultures. The expression of the putative myogenic candidates identified from the omics analyses was analysed by q-RT-PCR. Three distinct cultures were examined, initial fraction cells (1), CD34+/Sca1+ primary cells (used in omics analyses and efficient for myogenic differentiation) (2), and CD34+/Sca1+ cells after three passages (highly efficient for myogenic differentiation, S3 in Fig. 2C) (3). The data are expressed relative to *Gapdh* transcription (2^{-ΔCt}). b) Complement C3 is present in conditioned media. Purified human C3, control media (DMEM/decomplemented FCS) and conditioned media S3 (see Fig. 2C) were resolved in SDS-PAGE and analyzed by western blotting with anti-C3 antibodies. c) Growth media supplemented with serum (active complement) promoted myogenic differentiation. Initial fraction cells were cultured in DMEM/decomplemented FCS (Control), myogenic conditioned media S3 (Conditioned media S3), DMEM/decomplemented FCS supplemented with 1% mouse serum (Control + 1% mouse serum) and DMEM/decomplemented FCS supplemented with 1% mouse serum heated at 56°C (Control + 1% mouse serum + 56°C). After 3 days, myogenic differentiation was quantified and the control was indexed to 1. d) Complement C3 promoted myogenic differentiation. Primary cells were cultured in DMEM/decomplemented FCS (Control), and DMEM/decomplemented FCS supplemented with human C3 (Control + C3). Myogenic differentiation of the different cultures was assessed relative to the control indexed to 1. e) Culture media supplemented with C3 promote adipogenic differentiation. CD34+/Sca1+ cells were cultured in DMEM/decomplemented FCS (Control) and C3-supplemented control media (C3), respectively, for 6 days. Adipogenic differentiation was determined by Oil Red O staining, counterstained with DAPI. Bar scale corresponds to 100 µm. f) Myogenic and adipogenic differentiation reveal different kinetics. The differentiation of adipogenic and myogenic lineage cells was estimated relative cells cultured in control media g) C3 is internalized in myogenic and adipogenic cells. Fluorescently-labelled complement C3 was added to cultures of myogenic lineage cells (CD34 myogenic), and adipogenic lineage cells (Sca1 adipogenic), respectively, and observed microscopically after 4 h. Bar scale correspond to 20 µm. statistics: n=3, t-test P<0.001 (*).

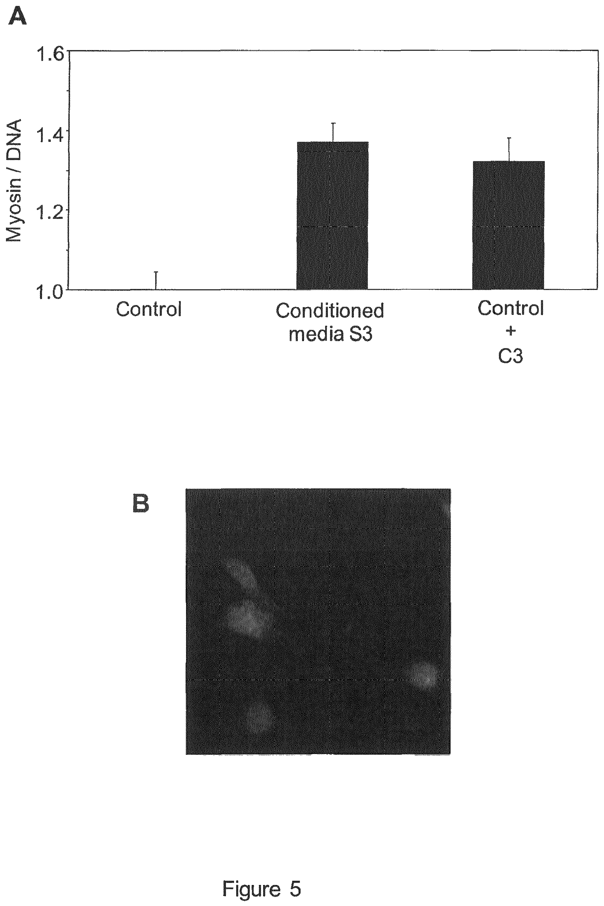

**Figure 5** **shows that myogenic differentiation of C2C12 cells is enhanced by internalization of C3. a)** C2C12 cells were cultured in DMEM-10%FCS (Control), conditioned media S3 (conditioned media S3), and control media supplemented with C3 (control + C3). Myogenic differentiation of the cultures was determined after 5 days relative to the control indexed to 1. **b)** Fluorescently labeled C3 was supplemented to the control media of C2C12 cell culture. After 4h, cells were observed by fluorescent microscopy.

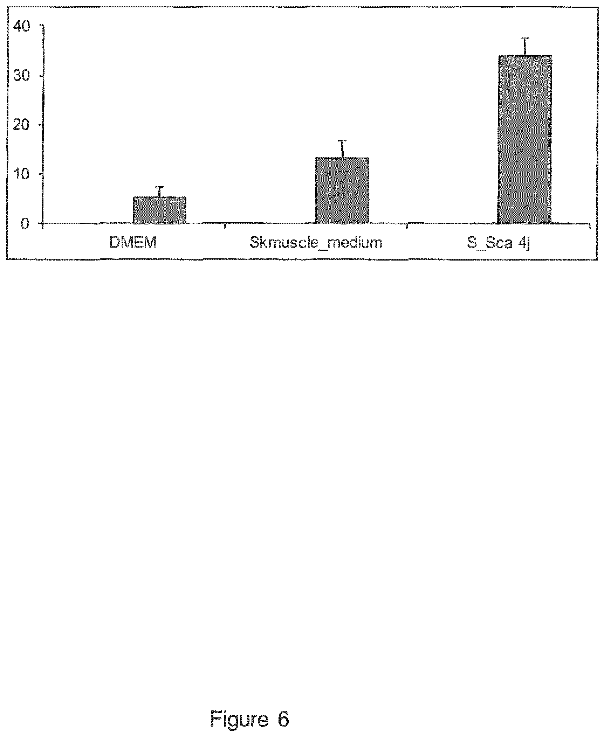

**Figure 6** **shows that preadipocytes conditioned medium comprising the C3 complement protein induces 2.5 times better the differentiation of myogenic progenitor cells into myogenic cells compared to a commercial medium.** myogenic progenitor cells were cultured in DMEM/decomplemented FCS (DMEM), supplemented with commercial medium lonza (Skmuscle medium) or with the preadipocytes conditioned medium comprising the C3 complement protein prepared by the inventors (S Sca 4j). Said preadipocytes conditioned medium (S Sca 4j) was prepared from the supernatant of 24h of foetal CD34+/Sca1+ cell culture at day 4. After four days the myogenic differentiation of the cultures was evaluated by quantifying myogenic nuclei (% in ordinate).

The present invention will be explained in detail with examples in the following text, but the technical scope of the present invention is not limited to these examples.

### EXAMPLES

### I. Material & Methods

### 1.1 Animals and isolation of foetal cells

All experiments were carried out in accordance with guide-lines approved by the University of Nantes, Institutional Animal Care and Use Committee. Adult swiss mice were maintained in our laboratory under controlled environmental conditions and fed with food and water *ad libidum.* The pregnant mice were sacrificed by cervical dislocation. Muscle cells were obtained from all four legs of 17-dpc mouse foetuses as previously described (Dupas, T. *et al*., 2011). One experiment is carried out from about 30 foetuses from two or three pregnant mice. Briefly muscles were removed from the legs, minced into small fragments and digested in collagenase I (Gibco) 0.1% in Dulbecco's modified Eagle's medium (DMEM) (Gibco) with 1% penicillin/streptomycin/fungizon (Gibco) and 0,05% fungizon (Gibco) for 1.5 h at 37°C. Upon sequential filtering through 70 µm and 40 µm cell strainers (BD-Falcon), the cells were collected by centrifugation at 350g and resuspended in DMEM/decomplemented fetal calf serum (10%) (DMEM/decomplemented FCS). Resulting cells corresponding to the initial fraction of the muscle cells were checked for viability using trypan blue exclusion dye and counted in a hematocytometer.

### I.2 Immunomagnetic-bead cell sorting

Cells were sorted by MACS according to the manufacturer's instructions (Miltenyi Biotech). For CD34+ sorting the freshly isolated cell suspensions were incubated for 12 min at 4°C with a biotinylated rat anti mouse CD34+ antibody (clone RAM34, eBioscience 13-0341-85) 1/50 in PBS/ EDTA (2mM)/ fetal calf serum (10%) (PEF) rinsed in PEF then magnetically labelled for 17 min at 4°C with anti-biotin-coated MicroBeads (1/25 in PEF) (Miltenyi Biotech 130-090-616). Then cells were rinsed twice in PEF and MACS was performed by passing the cells over cell-separation columns (Miltenyi Biotech 130-042-201). In order to obtain CD34+ cells subset depleted either CD31+ or Sca1+ cells, in a first step the freshly isolated cell suspensions were incubated with PE-conjugated rat anti-mouse CD31 (clone 390, Southern Biotech 1625-09) or PE-conjugated rat anti-mouse Sca1+ antibodies (clone D7, eBioscience 12-5981-83) diluted 1/50 and the CD31+ and Sca1+ cells were collected using anti-PE MicroBeads (Miltenyi Biotech 130-048-801). In a second step the negative fractions for CD31+ or Sca1+ were incubated with the biotinylated rat anti mouse CD34+ antibody and processed as described above to obtain CD34+/CD31- or CD34+/Sca1-cells populations. The cells of each subset were collected by centrifugation at 350g and resuspended in DMEM/decomplemented FCS and counted. The quality of the sorting is routinely examined by immunofluorescent microscopy.

### 1.3 Cell culture in standard conditions

Cell cultures were carried out in DMEM/decomplemented FCS and used as a control, otherwise indicated. The different cell subsets (foetal cells, CD34+, CD34+/CD31-, CD34+/Sca1- cells and foetal Sca1+ cells) were plated at 20,000 cells per cm2 either on 35 mm tissue culture treated dishes (Falcon 353001), on collagen-coated 8-chamber tissue culture glass slides (BD Falcon 3541108) or on collagen-coated 96 well cell culture plates (Greiner bio-one 655180). Cultures were maintained in a humidified atmosphere at 5% CO2 at 37°C.

### 1.4 Preparation of conditioned media

Foetal CD34+/Sca1+ cells were first grown in DMEM/decomplemented FCS on 35 mm dishes plated at 28,000 cells per cm2 from 2 days to 6 days and the culture medium was changed every day. In a first series of experiments the Sca1 conditioned media (S0) were prepared from the supernatant of 24 h of cell culture between day 1-2, day 2-3, day 3-4 and day 5-6. The fourth day of culture Sca1 cells were near confluence, at this stage a second series of experiments were done, cells were harvested using a trypsin digestion (trypsin-EDTA) then were grown on new dishes in DFCS in the same conditions as above. Conditioned media from this first passage (S1) were prepared from the supernatant of 24 h of cell culture between day 3-4. Then two successive passages were performed in a similar manner to collect S2 and S3 conditioned media. All the supernatants were filtered upon 0,22µm and frozen at -80°C.

### 1.5 Cells culture in conditioned media

Cells were directly plated at the same conditions as described for the commercial medium with the conditioned media S0 to S3.

### I.6 Determination of myogenic differentiation

Myogenic differentiation of the cells was determinate after 4 days of culture by immunochemical analyses. The cultures in 96-well plates or in 8-well slides were fixed for 10 min with 4% paraformaldehyde in PBS (PAF), rinsed in PBS, permeabilized with 5% Triton X-100 (Sigma) 1 h at 37°C, rinsed twice with PBS and then incubated overnight at 4°C with MY32, an Ab for myosin heavy chain (1/1000, Sigma), then revealed 1 h at room temperature by FITC-conjugated goat anti-mouse Ab (1/100, Clinisciences). Nuclei were counterstained with bis-benzimide. Quantitative analysis of myogenic differentiation were carried out by quantifying myosin with MY32 antibody and nuclei with DAPI staining in 96-well plates cultures using a fluorometer (POLARstar Omega - BMG labtech). Myogenic differentiation was expressed by the ratio of the FITC myosin signal / Bis-Benzimide DNA signal. Quantification was based on two to four independent experiments with at least four to six wells each. The percentages of myogenic nuclei were quantifying directly from microscopic observations (Nikon Ni-E microscope) by calculating the ratio of the nuclei within myosin expressing cells/the total number of nuclei/cm2 using ImageJ software (http://rsb.info.nih.gov/ij/). Two to four independent experiments with at least three to four wells were done and four microscopic fields per well were counted.

### 1.7 Determination of adipogenic differentiation

Cell cultures were rinsed 5 min in PBS, fixed with (PFA) for 20 min, washed three times with distilled water and stained in oil Red O solution for 30 min at 37°C and then washed five times in distilled water (Koopman, R. *et al*., 2001). Nuclei were counterstained with bis-benzimide. Quantification of adipogenic differentiation was done from microscopic determinations of the lipid accumulations by quantifying the areas stained by the red oil per cm2. The results were expressed as the ratio of lipid surface / number nuclei per cm². Two independent experiments with two wells were done and ten microscopic fields per well were counted.

### 1.8 RNA analyses

Total RNA was isolated using Trizol (Invitrogen) and the absence of DNA was determined by PCR. Reverse transcription reactions were performed using the iScript cDNA synthesis kit according to the manufacturer's instructions (Bio-Rad). RT-PCRs were performed using appropriate primer sets and the Maxima SYBR green qPCR master mix according to the manufacturer's instructions (Fermentas), and analyzed using Bio-Rad CFX manager (Bio-Rad) using *Gapdh* as a reference. Transcriptomic analyses were performed using an Agilent mouse expression array according to the manufacturer's instructions.

### I.9 Protein analyses

Electrophoretic analyses were carried out according to standard protocols. Pulse-labelling with tritiated lysine was performed for 24 h by adding 0.5% (v/v) of L-[4,5 ³H(N)]-Lysine (Specific Activity: 80-110 Ci/mmol) to DMEM/decomplemented FCS. Complement C3 was immunodetected using anti-C3 antibodies (Origen). Samples were prepared for mass spectrometry analyses as previously ⁵ with minor modifications. The culture medium was supplemented with 0.8 mM heavy Lysine and 0.4 mM AHA. Click chemistry was carried out using dibenzocyclooctyne (DBCO) magnetic beads according to the manufacturer's instructions (Jena Bioscience). The beads were then extensively washed with urea and SDS and the coupling of proteins to beads was examined by addition of fluorescein-5-maleimide and SDS-PAGE. Complement C3 was fluorescently labelled by mixing 0.1 nmol of purified human C3 (Teco Medical) with 2 nmol of DyLight 550 NHS Ester (Thermo Fisher) according to the manufacturer's instructions and excess dye was removed with spin columns (cut-off 5 k). The efficiency of the labelling was then evaluated by SDS-PAGE.

### I.10 Statistical analyses

For cell culture studies, at least three independent experiments were carried out. Microscopic analyses were performed at least in duplicate and at least ten random fields were imaged per sample. Fluorimetry analyses were performed at least on six culture wells. Data are presented as mean ± SEM. Differences between groups were tested for statistical significance an unpaired two-tailed Student's t test. P < 0.0001was considered statistically highly significant and P < 0.005 was considered statistically significant.

### II. Results

### II.1 Preadipocytes conditioned medium, but not adipocytes conditioned medium, induces the differentiation of myogenic progenitor cells into myogenic cells via secreted factors

CD34+ mouse foetal muscle cells exhibit muscle regeneration properties and are composed of three distinguishable lineages; myogenic, angiogenic (CD34+/CD31+) and adipogenic (CD34+/Sca1+) Dupas, T. *et al*., 2011; Auda-Boucher, G. *et al*., 2007). Using cell cultures, the inventors determined whether myogenic differentiation of CD34+ foetal muscle cells is affected by the absence of the angiogenic sub-population (CD34+/CD31+) and the adipogenic sub-population (CD34+/Sca1+) (Fig. 1). The inventors found that cultures with similar growths exhibited a significant decrease of the myogenic differentiation in absence of cells of the adipogenic lineage.

To determine whether molecules from adipogenic lineage stimulate the differentiation of myogenic precursor cells, conditioned media from adipogenic lineage cell cultures were prepared and assayed for effect on cells harvested from the foetal initial fraction (the initial fraction corresponds to individual cells harvested from muscle) (Fig. 2A, B,). Analyses of myogenic differentiation were performed by counting the percentage of myogenic nuclei. The results revealed an increase of myogenic nuclei when the initial fraction was cultured in the conditioned media prepared from a proliferative adipogenic lineage culture (Day1-2 to Day3-4 - corresponding to a preadipocytes conditioned medium). Beyond Day4-5, the CD34+/Sca1+ cell cultures reached confluence and began to differentiation into adipocyte and the resulting conditioned exhibited a decrease of myogenic nuclei (compare Day3-4 and Day5-6). These results suggested that myogenic effect of the conditioned media correlates with the proliferative and undifferentiated states of the CD34+/Sca1+ cells.

To verify that the proliferation of the undifferentiated CD34+/Sca1+ cells supported the myogenic effect, the inventors prepared conditioned media of cultured CD34+/Sca1+ and performed passages of the cells after 4 day-cultures, prior to reach confluence for allowing cell expansion (Fig. 2C). The analyses of the conditioned media prepared from the different passages showed that myogenic effects are recovered over three passages of the Sca1 cells, above this number of passages, CD34+/Sca1+ cells did not proliferate and myogenic effect was significantly reduced (data not shown). Unexpectedly, the inventors reproducibly noticed that the passages of the CD34+/Sca1+ cells improved the myogenic effects of the conditioned media. Supposedly, the passage of the CD34+/Sca1+ cells induced selection. However, because the myogenic effect might be due to different proliferation rates of the myogenic lineage, the inventors examined the kinetics of proliferation in presence of control media and conditioned media (data not shown). The results revealed that the cell proliferation is unaffected by the conditioned media as similar rates of proliferation were observed in control and conditioned media.

The inventors concluded that conditioned media from proliferative adipogenic lineage sustain myogenic differentiation effects. The inventors have thus shown that preadipocytes conditioned medium, but not adipocytes conditioned medium, induces the differentiation of myogenic progenitor cells into myogenic cells via secreted factors.

### II.2 Analyses of the preadipocytes secretome

To investigate the myogenic factors from pre-adipocytes, the inventors first attempted to determine their biochemical nature. Conditioned media were thus subjected to RNase treatment and heat prior to evaluating the residual myogenic activity. The conditioned media were unaffected by RNase treatment (data not shown), but were sensitive to heat treatment, suggesting that the myogenic factors are proteins (Fig. 3A). To confirm the presence of proteins from proliferative adipogenic lineage within the conditioned media (i.e. preadipocytes conditioned media), the cells were pulse-labelled with tritiated lysine and the culture media were examined by autoradiography (Fig. 3B). Only a limited number of proteins were radioactively labelled, suggesting that the myogenic factors from adipogenic lineage are secreted and did not result from cell lysis. These proteins were synthesized in low abundance as no specific bands could be detected by gel staining (data not shown). Next, the inventors investigated whether the myogenic factors from the adipogenic lineage recovered in the conditioned media were soluble or packaged in micro-vesicles, as described for the myoblast secretome (Le Bihan, M.C. *et al*., 2012). Conditioned media were spun at high speed to pellet micro-vesicles and the supernatant was assayed for its capacity to promote myogenic differentiation (Fig. 3C). Fluorecence of myosin/DNA of initial fraction cultured with the soluble fractions were similar to that with the conditioned media. These results showed that proteins of the conditioned media involved in myogenic differentiation are secreted from the proliferative adipogenic lineage (i.e. secreted from preadipocytes).

To identify the myogenic factors of the conditioned media, proteomic analyses of the secretome of the adipogenic lineage were carried out. Typically for such analyses, cells are grown in the absence of serum (Le Bihan, M.C. *et al*., 2012 and 2015). The inventors therefore verified that the cells of the adipogenic lineage proliferated and secreted the myogenic factors in the absence of serum. CD34+/Sca1+ cells were cultured in the presence and in the absence of decomplemented FCS, and the culture media were harvested to prepare the conditioned media as earlier. Then, the conditioned media were assayed for their ability to enhance myogenic differentiation of initial fraction cells (Fig. 3D). Consistent with our previous analyses, the inventors found that myogenic differentiation with conditioned media from CD34+/Sca1+ cells in presence of decomplemented FCS is about 2 fold greater than control cultures (see Fig. 2C, control vs SO). However, when decomplemented FCS was omitted CD34+/Sca1+ cell cultures, the resulting conditioned media failed to enhancement the myogenic differentiation. These results showed that CD34+/Sca1+ cells secreted myogenic factors when cultured in presence of decomplemented FCS, and thus proteomic analyses CD34+/Sca1+ cell secretome required FCS in the cell cultures. To overcome this limitation, the cells of adipogenic lineage were pulse-labelled with azidohomoalanine (AHA), a non-natural amino acid that can substitute for methionine, and concomitantly, non-radioactive heavy lysine was added to the culture media (Eichelbaum, K. *et al.* 2012). The secretome proteins were thus purified by coupling the AHA-containing proteins with DBCO magnetic beads. The covalently attached proteins were then analyzed by LC-MS/MS and the heavy lysine-containing proteins were selected (Fig. 3E, data not shown). Of the 90 proteins that were identified, only 28 were readily secreted from cells (data not shown).

To ensure that the identified proteins of the adipogenic lineage cell secretome were readily associated with the proliferative state of the cells, whereby the effect on myogenic differentiation has been observed, transcriptomic analyses were carried out (Fig. 3F). Adipogenic lineage cells in early and late proliferation were thus compared. 1,113 probes, corresponding to 749 genes, were up-regulated in late proliferation (data not shown). To narrow down rationally the number of genes that might be associated with myogenic differentiation enhancement, the expression of these 749 genes in late proliferation of adipogenic lineage cells was compared with that in angiogenic lineage cells, which have no effect on myogenic differentiation (see Fig. 1). Of the 749 genes, only 22 were up-regulated in the adipogenic lineage cells. Again, the inventors verified that gene products were secreted from cells and found that 17 gene products were present in the extracellular medium (data not shown).

The inventors reasoned that the factors involved in the enhancement of myogenic differentiation are probably highlighted in both proteomic and transcriptomic analyses. The proteins shared between the 28 proteins resulting from the proteomic analyses and the 17 gene products resulting from the transcriptomic analyses were thus determined. Eleven proteins met these criteria and could be putative candidates for myogenic differentiation (Fig. 3G).

### II.3 Complement protein C3 induces the differentiation of myogenic progenitor cells into myogenic cells

The inventors have shown that the secretome from adipogenic lineage cells preserves the myogenic effect as long as the secretory cells are proliferative (i.e. preadipocytes) and do not engage in adipogenic differentiation (see Fig. 2). The inventors thus reasoned that the factors involved in myogenic differentiation are synthesized in primary cells of adipogenic lineage and cells resulting from serial passages. Therefore, the expression of the genes encoding for the putative candidates involved in myogenic differentiation was examined (Fig. 4A, data not shown). Strikingly, the gene encoding for the third component of the complement system (C3), a key part of the innate immune system (Sahu, A. & Lambris, J.D., 2001), was expressed in the primary adipogenic lineage cells as well as in the proliferative cells following several passages. In contrast, all the other genes exhibited a drastically lower expression after the passages than the primary adipogenic lineage cells. The inventors then verified by western blotting that full length C3 was secreted into the conditioned media (Fig. 4B). Immunodetection using an antibody to human C3 revealed the presence of bands that are consistent with C3 and in the same molecular weight range as the band detected by pulse-labelled tritiated lysine (see Fig. 3B).

To confirm that C3 is involved in enhancing myogenic differentiation, the inventors examined whether supplementing culture media with C3 led to such an enhancement. As molecules of the complement are known to be abundant in serum, culture media were supplemented with 1% mouse serum and the effect on myogenic differentiation was examined (Fig. 4C). The analyses of the cell cultures revealed that the addition of mouse serum in the culture stimulated myogenic differentiation. Further evidence for a role of components of the complement in myogenic differentiation was revealed by the loss of the myogenic effect when mouse serum was heated to 56°C to inactivate the complement. To ensure that C3 is responsible for myogenic differentiation, the effect on myogenic differentiation of precursor cultures supplemented with purified C3 was examined (Fig. 4D). As C3 protein presents high homology between mouse and human, these experiments were carried out with human C3 (77% identity, 92.7% similar, in 1666 aa). Similarly, the efficiency of differentiation in an adult cell culture (C2C12 cell line) was greater when human C3 was present in culture media (Fig.5). These results demonstrate that the differentiation of myogenic progenitor cells into myogenic cells is enhanced by the complement protein C3.

Analyses of the effects of C3-containing media were also carried out directly on adipogenic lineage cells (Fig. 4E). CD34+/Sca1+ cells differentiated faster than control cultures with media supplemented with C3. Thus, C3 enhanced adipogenic differentiation. Comparison of the kinetics of differentiation of myogenic lineage cells and adipogenic lineage cells induced by C3 and relative to the respective controls revealed significantly faster myogenic differentiation than adipogenic differentiation (Fig. 4F). These observations suggested that in the muscle environment, wherein myogenic lineage and adipogenic lineage among other lineages, C3-induced differentiation promotes myogenesis faster than adipogenesis. Moreover, these results suggested a putative autocrine signalling mechanism, as C3 production by pre-adipocytes is down-regulated by the induction of adipogenic differentiation by C3 itself.

It is generally believed that cytokines induce cell differentiation through the activation of signalling pathways. We thus examined whether such pathway activation accounted for the effect of C3 on myogenic differentiation. Common signalling pathways were scanned by antibody arrays, which showed no activation of a specific signalling pathway by C3 (data not shown). It has been reported that C3 can activate immune cells by internalization (Tam, J.C. et *al*., 2014). Fluorescently-labelled C3 was added to culture media and its cellular location was determined by microscopy (Fig. 4G, data not shown). The inventors clearly observed that C3 molecules were rapidly internalized into the cytoplasm of myogenic CD34+ cells and adipogenic CD34+/Sca1+ cells. These results show that differentiation induced by the complement protein C3 involves its internalization into the cytoplasm.

### II.5 Preadipocytes conditioned medium comprising the C3 complement protein induces 2.5 times better the differentiation of myogenic progenitor cells into myogenic cells compared to a commercial medium

Myogenic progenitor cells were cultured in DMEM/decomplemented FCS (DMEM), supplemented with commercial medium lonza (Skmuscle medium) or with the preadipocytes conditioned medium comprising the C3 complement protein prepared by the inventors (S Sca 4j). Said preadipocytes conditioned medium (S Sca 4j) was prepared from the supernatant of 24h of foetal CD34+/Sca1+ cell culture at day 4. After four days the myogenic differentiation of the cultures was evaluated by quantifying myogenic nuclei.

As shown in Figure 6, the inventors have demonstrated that preadipocytes conditioned medium comprising the C3 complement protein induces 2.5 times better the differentiation of myogenic progenitor cells into myogenic cells compared to a commercial medium.

### BIBLIOGRAPHIC REFERENCES

Dupas, T. et al., Fetal muscle contains different CD34+ cell subsets that distinctly differentiate into adipogenic, angiogenic and myogenic lineages. Stem Cell Res 7 (3), 230-243 (2011).
Auda-Boucher, G. et al., Fetal muscle-derived cells can repair dystrophic muscles in mdx mice. Exp Cell Res 313 (5), 997-1007 (2007).
Le Bihan, M.C. et al., In-depth analysis of the secretome identifies three major independent secretory pathways in differentiating human myoblasts. J Proteomics 77, 344-356 (2012).
Le Bihan, M.C. et al., Cellular Proteome Dynamics during Differentiation of Human Primary Myoblasts. J Proteome Res 14 (8), 3348-3361 (2015).
Eichelbaum, K., Winter, M., Berriel Diaz, M., Herzig, S., & Krijgsveld, J., Selective enrichment of newly synthesized proteins for quantitative secretome analysis. Nat Biotechnol 30 (10), 984-990 (2012).
Sahu, A. & Lambris, J.D., Structure and biology of complement protein C3, a connecting link between innate and acquired immunity. Immunol Rev 180, 35-48 (2001).
Tam, J.C., Bidgood, S.R., McEwan, W.A., & James, L.C., Intracellular sensing of complement C3 activates cell autonomous immunity. Science 345 (6201), 1256070 (2014).
Koopman, R., Schaart, G., & Hesselink, M.K., Optimisation of oil red O staining permits combination with immunofluorescence and automated quantification of lipids. Histochem Cell Biol 116 (1), 63-68 (2001).

## Claims

1. An ex *vivo* method for inducing the differentiation of skeletal myogenic progenitor cells into skeletal myogenic cells, said method comprising the step of:
- culturing the skeletal myogenic progenitor cells in the presence of a complement protein C3.

2. The method according to claim 1, wherein said method further comprises the step of:
- transfecting the skeletal myogenic progenitor cells with an expression vector encoding a complement protein C3.

3. The method according to claim 1, wherein said complement protein C3 is comprised in a preadipocytes conditioned medium.

4. The method according to claim 1 or 3, wherein said complement protein C3 is comprised in a culture medium, which further comprises at least another compound selected from the group consisting of:
- antibiotics, amino-acids, vitamins, inorganics salts, carbohydrates, hormones, cytokines, growth factors and skeletal myogenic differentiation factors.

5. The method according to claim 4, wherein, in the culture medium, the concentration of said complement protein C3 ranges from 0,05 % to 2 % in particular from 0,25 % to 1,75 % and more particularly from 0,5 % to 1,25 % and even more particularly 1 %.

6. *Ex vivo* use of a complement protein C3 or a nucleic acid molecule encoding said protein as a skeletal myogenic differentiation factor.

7. Use of a complement protein C3 or a nucleic acid molecule encoding said protein for the manufacture of a skeletal myogenic differentiation product, in particular a skeletal myogenic differentiation medium.

8. *Ex vivo* use of an additive to a culture medium for inducing the differentiation of skeletal myogenic progenitor cells into skeletal myogenic cells, said additive comprising a complement protein C3.

9. The use according to claim 8, wherein said additive further comprises at least another compound selected from the group consisting of:
- antibiotics, amino-acids, vitamins, inorganics salts, carbohydrates, hormones, cytokines, growth factors and skeletal myogenic differentiation factors.

10. A skeletal myogenic differentiation medium comprising:
- a basal medium, in particular for culturing mammalian cells; and
- a complement C3 protein,
wherein said skeletal myogenic differentiation medium is complemented serum-free.

11. The skeletal myogenic differentiation medium according to claim 10, wherein said medium further comprising at least another compound selected from the group consisting of:
- antibiotics, hormones, cytokines, growth factors and skeletal myogenic differentiation factors.

12. A skeletal myogenic differentiation kit comprising:
- a basal medium, in particular for culturing mammalian cells; and
- an additive comprising a complement protein C3, wherein said additive is complemented serum-free.

13. *Ex vivo* use of the skeletal myogenic differentiation medium according to claim 10 or 11, or of the skeletal myogenic differentiation kit according to claim 12, for inducing the differentiation of skeletal myogenic progenitor cells into skeletal myogenic cells.

14. A complement protein C3 or an acid nucleic molecule encoding said protein for use as a medicament in the treatment and/or prevention of a skeletal muscle loss condition.

15. A complement protein C3 or an acid nucleic molecule encoding said protein according to claim 14, for use according to claim 14 wherein said skeletal muscle loss condition is selected from the group consisting of skeletal muscle degenerative diseases and skeletal muscular atrophy diseases.
